# EUROPEAN PATENT APPLICATION

(11) **EP 4 559 406 A1**
(43) Date of publication of application: **28.05.2025**
(21) Application number: 24214394.9
(22) Date of filing: 21.11.2024
(51) Int. Cl.: A61B 8/00, A61N 7/00

(54) **WEARABLE CLOSED LOOP TUS SYSTEM AND METHOD**

(30) Priority: 21.11.2023 US 202363601577 P; 18.10.2024 US 202418920615
(71) Applicant: Sanmai Technologies, PBC, Sunnyvale, CA 94087 (US)
(72) Inventor: DAFT, Christopher, Sunnyvale, CA 94087 (US); WU, Bicheng, Sunnyvale, CA 94087 (US)
(74) Representative: Araujo, Daniel

(57) **Abstract**

A TUS (transcranial ultrasound) system with a matrix array transducer in a wearable format is disclosed. The TUS system uses ASIC (application-specific integrated circuit) and MEMS (micro-electromechanical system) technologies to achieve power, size, and weight reductions, allowing the device to be worn. In an embodiment, the TUS system can reliably find a target anatomical structure and remain locked onto the target throughout usage. All real-time tasks are controlled locally within the TUS system for a closed-loop operation. Safeguards enable subjects to use the system at home for medical treatment and wellness usage, as well as in a clinic. A method for using the system is disclosed.

## Description

### TECHNICAL FIELD

This invention relates to transcranial ultrasound (TUS) systems comprising a wearable wellness device suitable for clinical and for non-therapeutical purposes.

### PRIOR ART

TUS systems help treat several types of mental illness, but these systems do not guarantee that the ultrasound stimulation accurately reaches the anatomical targets. The skull's curvature and its variation in thickness obscure the ultrasound (US) delivery to the desired location, especially with small and deep targets such as the amygdala. This causes known TUS systems to be under-optimized in terms of targeting accuracy (i.e. the ability to target specific locations with the TUS system with efficacy) and/or energy consumption (e.g. reducing energy inefficiently, i.e. the amount of energy consumed without achieving effective results). Thus, there is a need to optimize the operation of such TUS systems in order to improve reduce their energy consumption while ensuring optimal operation of the TUS system.

Matrix array systems can solve the problem of poor targeting by acquiring volume data sets of brain anatomy, wherein this improved targeting accuracy also provides increased safety when using the TUS system, as the risk of targeting wrong anatomical areas that could cause harm to the user can be avoided or significantly reduced. Current solutions are targeted for clinically controlled usage, where a clinician defines the treatment for an illness. There is a need to expand the capabilities of TUS systems as a wellness device for individuals who want to improve their well-being, even if they do not have any medical diagnosis. Additionally, at-home medical treatment controlled by a physician has cost and convenience advantages over in-clinic procedures. For this, the TUS system needs to be in a wearable format. In addition to ensuring that wearable TUS systems correctly target the correct anatomical target safely, large cost, power, size, and weight reductions are required for the system to be in a wearable form factor.

### SUMMARY OF THE INVENTION

A first aspect of the invention refers to a system (e.g. a TUS system) comprising a wearable device (also referred to as TUS device) configured as a headband device (also referred to as TUS headband) and also comprising a secure network device.

The headband device comprises one or more transcranial ultrasound (TUS) tiles, wherein each of these TUS tiles comprises a plurality of transducer elements (preferably configured as matrix array transducers) connected to a respective integrated circuit. Preferably, the TUS tiles may be configured as a matrix of TUS tiles, i.e. a two-dimensional array of TUS tiles. Therefore, in the context of the present invention, a TUS tile refers to one of a plurality of small functional blocks comprising a plurality of ultrasound transducer elements (preferably arranged forming an array) and at least one integrated circuit connected to said plurality of ultrasound transducer elements. Ultrasound transducer elements may be matrix array transducers, wherein the individual elements of the matrix array may be configured to operate as transmit, receive, or as both transmit and receive elements. Each TUS tile may be configured to emit and receive ultrasound waves. The integrated circuit may be configured as an interface for allowing bidirectional communication between the plurality of ultrasound transducer elements and the rest of the TUS system (e.g. the integrated circuits may be configured to generate a beamform transmit based on a signal specification provided by the secure network device before reaching the transducers; and/or may be configured to manage any signal received from the transducers to transmit it to the rest of the TUS system).

The secure network device is configured to be in communication with the headband device. The secure network device is configured for generating a signal specification (e.g. "start', "stop", target locations, treatment details, and/or stimulation parameters -e.g. parameters for characterizing an output of the headband device in any operation mode) and transmitting the signal specification to the headband device, wherein the headband device is configured to operate in a stimulation mode in which the headband device is configured to output a stimulation signal beam (e.g. an ultrasound stimulation signal; also referred to as stimulation beam) based on the signal specification via the one or more TUS tiles. The secure network device may be configured to be integrated in the wearable device or may be configured to be external (e.g. configured as a physically independent entity) to the wearable device. In those embodiments in which the secure network device is configured to be external to the wearable/headband device, the secure network device may preferably be configured to be in wireless communication (e.g. using a wireless network, such as a Wi-Fi or a Bluetooth wireless network) with the wearable/headband device.

For the purpose of achieving power, size, and weight reductions, allowing the headband device to be worn, the integrated circuit of each TUS tile may be configured as an ASIC (application-specific integrated circuit), and/or the plurality of transducers of each TUS tile may be configured as a MEMS (micro-electromechanical system) device. The MEMS devices of any of the embodiments of the invention may be configured as (or comprise) a MEMS die. Preferably, the plurality of transducer elements of each MEMS device may comprise at least one of a Piezoelectric Micromachined Ultrasound Transducer (pMUT) or a Capacitive Micromachined Ultrasonic Transducers (cMUT) or a bulk piezoelectric transducer.

Further, in any of the embodiments of the invention, each TUS tile may be configured such that the respective MEMS device/die may be arranged on top of stack (e.g. to sit atop) the ASIC, wherein the respective MEMS device/die, and the respective ASIC may be interconnected to each other with vertical interconnects. The output (acoustic ultrasound output) of the MEMS device/die may come from its backside (i.e. top of the stack). The MEMS device/die may be inverted to allow for vertical interconnects with the ASIC.

Also in a broadly compatible manner, each MEMS device may comprise at least one, preferably four, receive portions and a transmit portion. The transmit portion may be configured to be electrically separated from the at least one receive portion. A transmit portion may correspond to an individual transducer element of a matrix array transducers, said individual element being configured as a transmitter element (i.e. configured to transmit). Each of the at least one receive portion may correspond to an individual transducer element of a matrix array transducers, said individual element being configured as a receptor element (i.e. configured to receive). In some embodiments, the headband device may comprise at least one physiological measurement subsystem configured to monitor an efficacy of the system (e.g. by measuring a physiological reaction in a user, for example, in a body of the user, preferably in the head of the user). The system of the first aspect of the invention may be configured to control a timing of the stimulation signal beam based on the measurements (e.g. physiological data -e.g. biomarkers- related to a reaction in a body of a user to the stimulation signal) obtained by means of the at least one physiological measurement subsystem. In other words, the at least one physiological measurement system may be configured to measure physiological data of the user while using the TUS system, thereby allowing the TUS system to obtain information on the efficacy of the TUS system, which allows the TUS system to control/adapt a timing of the stimulation signal beam based on the measurements of the at least one physiological measurement system. The TUS system may be configured to operate in a closed loop (e.g. when operating in the stimulation mode) in which the timing of the stimulation signal beam may be continuously controlled based on the measurements of the at least one physiological measurement subsystem. Preferably, the at least one physiological measurement subsystem may comprise one or more subsystems, each of which may be configured to obtain measurements in the form of one of: an electroencephalogram (EEG) measurement subsystem, an electrooculogram (EOG) measurement subsystem, an electromyography (EMG) measurement subsystem or an electrocardiogram (ECG) measurement subsystem. In preferred embodiments, the headband device may comprise at least one electroencephalogram (EEG) subsystem and preferably one or more of the aforementioned list of subsystem (e.g. EOG, EMG or ECG),

The TUS system may further comprise a secure network device configured such that all communication with the TUS system may be conducted through the secure network device. The secure network device may be configured to perform (e.g. on behalf of the wearable/headband device) at least one of authentication and encryption of all communications with the wearable/headband device, tamper detection and protection against side-channel attacks. The secure network device may comprise a microprocessor configured to control communications of the TUS system.

The TUS system may further comprise a real-time control circuit (also referred to as real-time controller or RT control) and a memory, which may comprise beam parameters for configuring the stimulation signal beam (e.g. the stimulation signal beam may be configured as a beam - e.g. an ultrasound beam- based on the signal specification provided by the secure network device but also based on the application of the beam parameters). The real-time control circuit may be configured to be coupled to the headband device and also may be configured to read beam parameters from the memory, to allow the TUS tiles to output a stimulation signal beam having a beam according to the beam parameters. The beam parameters may be arranged as a plurality of sets of beam parameters (e.g. each set being suitable for specific functionalities, such as operating in a particular operation mode - e.g. the stimulation mode, or the channel mode, or the beam mode- and/or onto a specific target). Each set of beam parameters may be configured as a list of beam parameters (e.g. beam list). In some embodiments, the memory may be part of the wearable/headband device. To maintain high security, the secure network device may be configured such that a user of the system is not allowed install any software on the secure network device. Secure network device may be configured to allow only safe commands to be introduced into the system to ensure the safe use of wearable device and the safety of the user. The real-time control circuit may be configured as Field-Programmable Gate Array (FPGA) which is a reconfigurable (e.g. reprogrammable) type of integrated circuit.

The real-time control circuit may be further configured to cause the headband device to operate in a channel mode, in which the TUS tiles are configured to obtain skull characterization data of a user by measuring an effect (e.g. a diminishment on the transmission capability) on ultrasound transmission caused by the skull (i.e. the particular characteristics) of a user. Human skulls vary in thickness, sound speed, and curvature which may cause a distorting ultrasound delivery by the TUS tiles (e.g. as the stimulation signal beam) onto some possible targets through attenuation, refraction, diffraction, and reflection. Channel mode may be used to characterize the skull's effect on ultrasound transmission for all ultrasound transducer elements of the TUS tiles. The results from this operation may be subsequently used to compensate for particular effects of a given skull characterization for all ultrasound transducer elements of the TUS tiles. Thus, in preferred embodiments, the secure network device may be further configured to generate skull compensation parameters based on the skull characterization data. The skull compensation parameters may be subsequently used for modifying an output of the TUS tiles (e.g. an output in the stimulation mode and/or in the beam mode). Preferably, the skull compensation parameters may be configured as time delay corrections and/or signal amplitude corrections for the output of the TUS tiles.

In some embodiments, the headband device may be configured to operate in a beam mode. The real-time control circuit may be configured to cause the headband device to operate in the beam mode. When operating in the beam mode, the headband device is configured for mapping (e.g. identifying) locations of brain structures of a user (e.g. different brain parts, blood flow locations and/or blood vessel locations) by means of the TUS tiles, thereby obtaining anatomy characterization data. In some embodiments, the TUS tiles may be configured to operate in the beam mode according to specific beam parameters (e.g. to generate specific waveforms as output of the TUS tiles) and/or may also operate applying the skull compensation parameters Thus, the skull compensation parameters generated in the channel mode may help to obtain a more precise determination of the locations of the brain structures when operating in the beam mode. The anatomy characterization data may comprise information of the anatomical configuration of the skull of the user (e.g. as a map or a model/representation of the skull). This anatomical characterization data may be used, for example: to avoid critical brain structures or blood vessels that may be affected the ultrasound stimulation; and/or to determine if the TUS headset is correctly positioned for targeting required anatomical regions before and during a stimulation session or treatment (e.g. if the user moves during a stimulation or treatment session). The obtention of the anatomy characterization data allows the TUS system to operate with improved efficacy and efficiency. The headband device may be configured to firstly obtain anatomy characterization data in the form of a volume greyscale image (2D image) of the brain structures, and subsequently obtain anatomy characterization data in the form of volume vessel data (e.g. blood flow), for example, by Doppler processing.

The TUS system may be further configured such that, when the headband device operates in the channel mode or in the beam mode, the real-time control circuit causes each of the TUS tiles to communicate with adjacent TUS tiles (especially when the TUS tiles are arranged in an array or matrix of TUS tiles). Preferably, the TUS tiles may be configured to be connectable in a chained-connection in which the TUS tiles are sequentially connected to each other (e.g. one after another from a first TUS tile to a last/final TUS tile), such that the integrated circuit (e.g. configured as an ASIC) of each TUS tile may be configured to receive data from its respective plurality of transducers (i.e. those belonging to the same TUS tile that the respective integrated circuit) and to transmit this data to the integrated circuit of a next TUS tile in the chained-connection. The integrated circuit of a TUS tile arranged as a final TUS tile in the chained-connection may be configured to provide a summation of the data generated by all the TUS tiles and transmit this summation to the real-time control circuit. The data generated by each TUS tile may related to the skull characterization data (e.g. may correspond to measurements made by the TUS tiles to generate the skull characterization data) in the channel mode, and may related to anatomy characterization data (e.g. may correspond to measurements made by the TUS tiles to obtain the anatomy characterization data). Further, in this same configuration the real-time control circuit may be configured to control/command the secure network device to stop transmitting the signal specification to the headband device.

In some embodiments, the TUS system may further include algorithmic or machine learning functions configured to optimise the operation of the TUS system. For example, the TUS system may use these capabilities to: improve the management of the physiological data measured by the at least one physiological subsystem to improve the efficacy of the TUS system; to generate more accurate skull compensation parameters; to obtain improve the reliability of the anatomy characterization data; and/or to improve the beam parameters to better perform in the different operating modes of the headband device.

In some embodiments, the TUS system may further comprise or may be connectable to a cloud server (which may be a server external to the system). The cloud server may be advantageously combined with the aforementioned machine learning functions to allow on cloud computation, which enhances the computing performance.

Preferably, the TUS system may be further configured such that, when the headband device operates in the stimulation mode, the real-time control circuit may be configured to cause the headband device to adapt the stimulation signal beam (which is provided as an output of the TUS tiles) based on the anatomy characterization data (e.g. the anatomy characterization data may be used by the TUS system to modify the stimulation signal specification and/or to select specific subsets of beam parameters compatible with the anatomy characterization data obtained). Both the channel mode and the beam mode, considered in independently or in combination, enhance the overall effectiveness of the TUS system for a wider range of users.

The TUS system may be configured to operate in a closed loop in which the TUS system operates in the channel mode and/or in the beam mode to continuously update the headband device knowledge of the location of the stimulation target or targets. In some embodiments, the TUS system may be configured such that the headband device sequentially operates in the channel mode, in the beam mode and in the stimulation mode in a closed loop, such that the TUS system is enabled to continuously update the headband device knowledge of the location of the stimulation targets. Further, this closed loop is also compatible with configuring the headband device to apply a closed loop when operating in the stimulation mode in which the timing of the stimulation signal beam may be continuously controlled based on the measurements of the at least one physiological measurement subsystem. In other words, the head band device may be configured to: continuously update the skull compensation data to generate updated skull compensation parameters in the channel mode; continuously update the anatomy characterization data in the beam mode; and continuously adapt the stimulation signal beam based on the updated anatomy characterization data. This close loop operation allows the TUS system to reliably find a target (e.g. an anatomical structure) and remain locked onto the target during the operation of the headband device (e.g. in the operation modes).

The integrated circuit of each TUS tile may comprise an analog block and a digital block. This configuration is broadly compatible with all the embodiments of the invention. Preferably, each integrated circuit may be configured as an ASIC.

The analog block may be configured to receive data receive data measured by the at least one receive portion (e.g. data related to any measurement or transduction made by the respective MEMS device; e.g. skull characterization data and/or data related to the locations of brain structures -i.e. anatomy characterization data-) of the respective MEMS device and to communicate said data to the digital block; and to receive output data from the digital block (e.g. data comprising instructions such as parameters or waveform characterization defining an output beam that the respective transmit portion of the MEMS device is required to provide; this data is also referred to as beam formation data) and transmit said output data to the transmit portion of the MEMS device, such that the transmit portion provides an output of the respective TUS tile further based on the output data.

The analog block of each integrated circuit may comprise: at least one analog receiving block and/or an analog transmit block. Preferably, the analog block may comprise a respective analog receiving block for each receiving portion of the MEMS device. Each analog receiving block may comprise at least one of a LNA configured to receive the data measured by the respective receive portion (i.e. by one of them), two mixers, two summers, two sigma-delta ADC in communication with the digital block. The analog transmit block may be configured to receive the output data from the digital block and to transmit said output data to the transmit section of the respective MEMS device. The analog transmit block may comprise a level shifter and/or an output stage configured to transmit the output data to the transmit section of the respective MEMS device.

In some embodiments, the TUS system may be configured to reliably find a target and remain locked onto the target throughout usage, wherein this may be achieved by configuring the headband device to operate in the aforementioned closed loop in which the headband device sequentially operates in the channel mode (wherein skull compensation parameters may be updated/modified based on the skull characterization data), in the beam mode (wherein anatomy characterization data is updated) and in the stimulation mode. The TUS system may be configured to send and receive sound (ultrasound waves) in various directions, sequentially creating "beams". The beams maybe controlled by a set of parameters stored in the local memory (e.g., in a table format) and are referred to in this application as "beam lists."

In some embodiments, the system may further comprise subject device configured to allow an interaction of the user/subject with the TUS headband device. The subject device may be configured as user interface device for allowing an interaction between the user and the headband device, and may preferably be configured as a smartphone, a tablet or a computer. In some embodiments, the TUS system may be configured such that, upon receiving a "start" signal (from the user via the subject device, e.g. via an app operating on the subject device such as a smartphone), all real-time tasks (e.g. those under the control of the real-time control circuit, such that the operation in the channel mode, the beam mode and the stimulation mode) can be controlled locally within the headband device (e.g.., in a closed-loop operation). The TUS system may comprise safeguards in the system configured to ensure that the TUS system can be used at home or in a clinic under a protocol defined by a clinician. In some embodiments, the TUS system may be used by a subject/user who does not have any medical diagnosis, just for the purpose of improving their well-being. The TUS system may comprise a variety of wellness preset protocols (e.g. beam parameters and/or stimulation signal specifications) are available to improve the wellness of a subject.

The term "drum" refers to individual MEMS structures with vibrating suspended membranes that are actuated by piezoelectric thin films.

The term "element" refers to an autonomously acting acoustic structure composed of several "drums." All the drums in an element are wired in parallel within the MEMS die. The ASIC electronics connects to the whole element, not to individual drums.

The term "cell" refers to a group of "elements" whose electronics are repeated through an ASIC die. In one implementation, a cell controls the operation of five elements, comprising one transmitter and four receivers.

As previously discussed, in some embodiments, the TUS system may be configured to operate in three modes: channel, beam, and stim (also referred to as stimulation mode). Channel mode of operation is suitable for skull characterization based on the skull's effect on ultrasound transmission for all ultrasound transducer elements. The results from this operation may be used to compensate for the effects of the skull for all ultrasound transducer elements. Beam mode operation is suitable for mapping and targeting brain structures and anatomy characterization. In some embodiments, corrected element delays and amplitudes acquired in channel mode ensure the elements correctly target the desired anatomical structures. Stim (or stimulation) mode is suitable for effectively applying a stimulation signal beam to a user. Table 1 below summarizes some functional aspects of the three modes of operation (channel, beam and stimulation). Column "TX beamformer operating?" specifies if the integrated circuit (e.g. the digital block of each integrated circuit) of each TUS tile is controlling the transmit sections/portions of the transducers to operate in transmit (TX) beamforming configuration. Column "RX signal chain operating?" indicates, for each given mode, if the TUS tiles (e.g. the integrated circuits) are connected to each other forming a chained connection to accumulatively measure/receive data (e.g. data related to skull characterization data and/or anatomy characterization data). Column "RX beamformer operating?" indicates integrated circuit (e.g. the digital block of each integrated circuit) of each TUS tile is controlling the receive sections/portions of the transducers are configured to operate in receive (RX) beamforming configuration. Also, column "Transmit Waveform" described the type of output provided by the TUS tiles (e.g. by the transmit sections/portions of each TUS tile) in each mode:

**Table 1**

| **Mode** | **TX beamformer operating?** | **RX signal chain operating?** | **RX beamformer operating?** | **Transmit Waveform** | **Usage** |
|---|---|---|---|---|---|
| Channel | No | Yes | No | Short pulse centered at the highest operating frequency | For skull characterization |
| Beam | Yes | Yes | Yes | For grayscale, a short pulse. | For mapping and targeting brain structures and anatomy characterization |
| | | | | For mapping of cranial vessels, 4-16 cycle tone-burst. | |
| Stim | Yes | No | No | Long tone-burst (300-5000 cycles). | For applying a stimulation signal beam |

There may be differences between the transmit and receive beamforming operations in Beam mode. These differences mean that different beam parameters (e.g.in the Beam List) may be used to control them, as detailed below:
- In the receive beamformer operation, time delays may be applied to the channel data continuously so that an image is in focus at every point (a dynamic receive focus). As depth increases for each channel, the schedule of delay variation is parameterized according to different beam parameters (e.g. in the Beam List).
- Received data from all transducer elements may be added together after appropriate delays are applied. This coherent summation may occur across TUS tiles by "chaining" the tiles together (e.g. by connecting the integrated circuits of adjacent tiles to each other).

- In the transmit beamformer, in the simplest mode of operation, there may be a single set of delays, one for each channel, which creates a focus at a fixed depth.
- More advanced transmit beam formation for guidance may involve the transmission of plane waves at differing angles, data from which can be retrospectively combined to produce a dynamic transmit focus. In this confocal system, the transmitter and receiver may be configured to be both in focus at each point in the image.

The digital block may be configured to have one or more of the following functions:
- Provision of "dynamic" delays to the received signals of the TUS tiles, that is, delays that change with the depth the sound is returning from. This delay may take into consideration how groups of elements have been combined in analog. The delay may be applied in coarse and fine stages. In one implementation, CORDIC (coordinate rotation digital computer) rotation may be used for fine delays. A decimation filter may be used in a different implementation, where the start and stop addresses are adjusted.
- Control of each channel's signal amplitude as a function of depth. Thus, the digital block may be configured to control an amplitude of each of the signals provided by each receive portion and/or each transmit portion of a MEMS cell/device (e.g. each of these signals representing a channel).
- Summation of channel data after the above delay and amplitude processing.
- Provision for adjustment of delay and amplitude (e.g. skull compensation parameters) to compensate for the aberrating effects of the skull.
- Ability to accept digital data from another ASIC, delay it, and sum with the beamformed data produced from the received elements. This allows tiles to be "chained."
- Creation of digital signals with the correct timing for each analog transmit block/section.

These may be delayed from the start signal by an amount with a geometrical component and a correction component based on skull measurements.
- Ability to operate in (1) normal beamforming mode, where channels are combined after delay and amplitude adjustment, (2) a channel mode, where data is not beamformed. This "channel" mode helps characterize the skull, and (3) in the stim mode.

A second aspect of the invention refers to a method of optimizing (also referred to as method of operating) a TUS system, which is preferably configured as a TUS system according to any of the embodiments of the first aspect of the invention, the method comprising:
placing a headband device comprising a plurality of TUS tiles on a subject scalp, the headband device being part of a TUS system;
obtaining beam parameters from a memory, the beam parameters specifying an operational mode of the TUS tiles;
obtaining skull characterization data by measuring with the TUS tiles an effect (e.g. a negative effect affecting on the efficiency of the headband device) on ultrasound transmission caused by the skull of the subject;
calculating skull compensation parameters based on the skull characterization data;
adjusting the TUS parameters based on the skull compensation parameters;
generating a stimulation signal based on the adjusted TUS parameters; and
outputting the stimulation signal by means of the TUS tiles of the headband device.

The method may further comprise obtaining anatomy characterization data (e.g. using a channel mode of the headband device) by generating volume grayscale data or vessel data by means of the TUS tiles to visualize a skull or blood vessel. Preferably, the method may further comprise verifying a positioning of the headband device based on the volume grayscale data or vessel data.

In preferred embodiments, the method may further comprise emitting the stimulation signal in response to verifying positioning of the headband device; and/or determining, based on the volume grayscale data or vessel data, whether relative motion exceeding a threshold between the headband device and the subject scalp occurred and generating a message to reposition the headband device in response to the determination.

The method of the second aspect of the invention may be carried out by the TUS system for non-medical uses, such as wellness applications (e.g. to relax and/or improved comfort/wellbeing). The TUS system according to any of the embodiments of the first aspect of the invention may be configured to be used in non-medical uses.

### BRIEF DESCRIPTION OF THE FIGURES

Fig. 1 shows a wearable TUS system 100 as per an embodiment.
Fig. 2 is an exemplary block diagram of TUS wearable 120 used in System 100.
Fig. 3 shows an example of tile 240 comprising PMUT MEMS array 250 and ASIC 260.
Fig. 4 shows an example ASIC 260 layout organized in a 16x8 grid.
Fig. 5 shows an example layout of one cell of MEMS 250 die.
Fig. 6 shows an exemplary block diagram of ASIC 260 as per an embodiment.
Fig. 7 shows an exemplary block diagram of Analog block 610 in ASIC 260.
Fig. 8 shows an exemplary functional block diagram of the digital block 620.
Fig. 9 shows an exemplary block diagram of DWE 810.
Fig. 10 is an exemplary method 1000 for closed loop operation of TUS wearable 120.

### DETAILED DESCRIPTION OF THE DRAWINGS

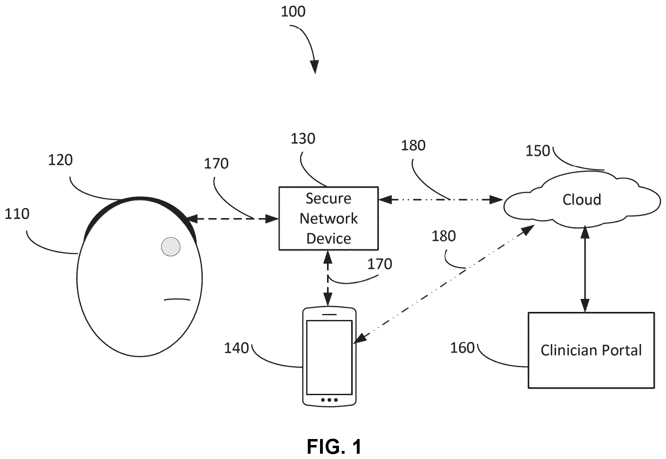

Fig. 1 depicts a TUS system comprising a TUS headband 120 (also referred to as headband device 120), a networking device 130 in communication with the TUS headband 120 0. In addition, the TUS system 100 depicted in Fig. 1 comprises an optional subject device 140 configured to allow an interaction of a user with the TUS system, and also comprises an optional cloud server 150, which is exemplary represented in connection with a clinician portal 160.

The TUS headband 120 (or TUS device) is shown arranged at a suitable location of a subject 110, ensuring proper contact with subject 110's scalp. Other wearable form factors such as a helmet to cover most of the scalp, half-helmet, half-headband, flexible cap, and visors are possible.

TUS system 100 includes a secure network device 130. All communication with the TUS system is through the secure network device 130. This allows for authentication and encryption of all communications, tamper detection, protection against side-channel attacks, and isolation of the subject device 140 and the internet from the operation of the TUS wearable 120. Headband 120 is configured to communicate with the secure network device 130 using a wireless 170 network such as Wi-Fi or Bluetooth in the preferred embodiment. A wired network (Ethernet, USB, etc.) may be used for communication in a different embodiment. To maintain high security, the TUS system is configured such that subject 110 (e.g. a user or unauthorized entity) cannot install any software on the secure network device 130. The secure network device 130 is configured to accept only safe commands into system 100 to ensure the safe use of the TUS headband 120 and the safety of subject 110. The network device 130 is shown as being optionally configured to communicate with the subject device 140, such as a smartphone, tablet, laptop, smartwatch, computer, etc., using a wireless protocol such as Wi-Fi or Bluetooth. The subject device 140 is configured as an interface for allowing the subject 110 to control (e.g. to interact with) the operation of the TUS system 100 (e.g. by using an app on the subject device 140). The subject device 140 may be configured to display (e.g. via an app) messages or other communications (data) from TUS system 100. Both the network device 130 and the subject device 140 are shown as being optionally connected to an optional cloud server 150 via Internet 180, allowing for authentication of network device 130 and subject device 140. The optional cloud Server 150 enables non-real-time aspects of system 100, such as updates to the system's machine learning aspects, management of subject data, and over-the-air software updates. The TUS system 100 of Fig. 1 is further depicted as comprising an optional clinician using portal 160 on server 150 may monitor the treatment and alter its course. It is noted that one of the aims the TUS system 100 is to provide an optimized setting and/or headband placement (e.g. based on the measurement of physiological data in a user body; and/or by determining specific skull compensation parameters; and/or by obtaining anatomy characterization data) which helps increase the efficacy of the TUS system (e.g. minimizing an energy required by the TUS system 100 and/or improving targeting accuracy) while ensuring an effective action of the headband device 120 (e.g. with an increased safety). Although not visible in Fig. 1, the TUS headband 120 of Fig. 1 comprises one or more transcranial ultrasound (TUS) tiles, wherein each of these TUS tiles comprises a plurality of transducer elements (e.g. ultrasound transducer elements) connected to a respective integrated circuit. Preferably, the TUS tiles may be configured as a matrix of TUS tiles (e.g. such that the TUS tiles are arranged adjacently), i.e. a two-dimensional array of transducer elements. Each TUS tile may be configured to emit and receive ultrasound. In addition to the ultrasound transducers, the headband 120 may include at least one physiological measurement subsystem configured to monitor an efficacy of the system (e.g. by measuring a physiological reaction in a user, for example, in a body of the user (e.g. an EEG sub-system, an EOG sub-system, EMG sub-system, an ECG sub-system). The physiological measurement subsystem allows for monitoring the physiological efficacy of the system, such as the effect on the brain. In preferred embodiments, the physiological measurement subsystem includes at least an EEG sub-system as part of the headband device 120. Subject device 140 receives data from the at least one physiological measurement subsystem (e.g. EEG sub-system) and RX data. System 100 includes at least one physiological measurement capable system such as EOG (electrooculogram), EMG (Electromyography), or ECG (electrocardiogram) in a different implementation.

In an implementation, the secure network device 130 may include an optional microprocessor 290 supporting the necessary wireless protocols 295 (Wi-Fi, Bluetooth, etc.). The microprocessor may be configured to translate high-level instructions from subject device 140 or cloud server 150 into low-level commands to the TUS device or headband 120. The instruction could include commands such as "start," "stop," target locations, treatment details, stimulation parameters, etc. Other non-real-time tasks may be managed by the secure network device 130. These tasks include authentication, encryption, tamper detection, protection against side-channel attacks, etc. An ASIC, off-the-shelf processor, or FPGA may be used for implementing the secure network device 130. In a different implementation, the functionality of the device 130 may be merged into the TUS headband 120.

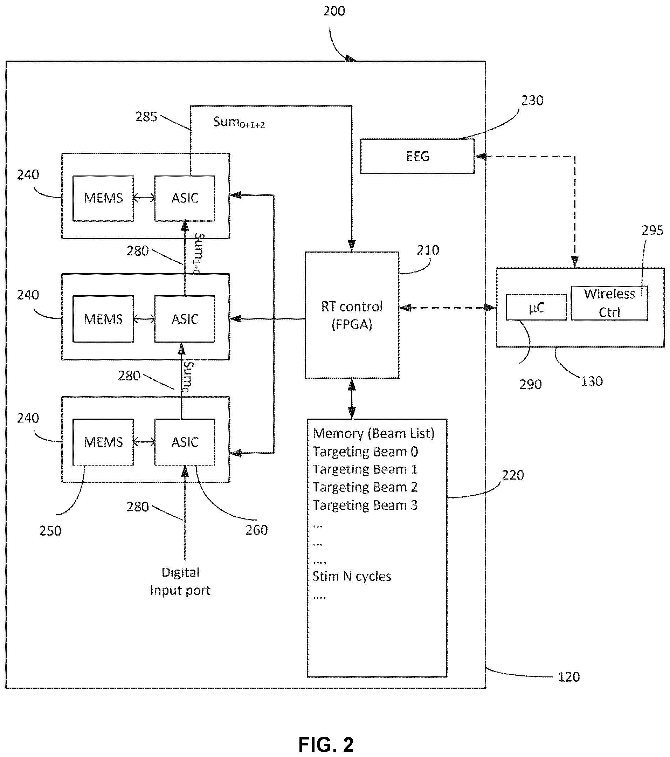

Fig. 2 is an exemplary block diagram 200 of TUS headband120 compatible with a TUS system 100 according to the first aspect of the invention. Referring to Fig. 2, TUS headband device 120 comprises a real-time (RT) control 210. Fig. 2 shows a preferred embodiment in which the RT control 210 is implemented using an FPGA. Other implementations are possible, where RT control 210 may be implemented using ASIC, microprocessors, etc. RT control 210 is preferably configured to communicate with the secure network device 130 using a wireless protocol such as Wi-Fi or Bluetooth or a wired interface such as USB. In some embodiments, FPGA 210 or microcontroller 290 may perform encryption and decryption.

The TUS headband 120 of Fig. 2 comprises a number of TUS tiles 240 attached to the RT control 210. Each TUS tile 240 comprises MEMS device 250 (or MEMS die) and an integrated circuit configured as an ASIC 260 (ASIC die or IC die). The MEMS devices 250 are preferably configured as a two-dimensional pMUT (Piezoelectric Micromachined Ultrasound Transducer) array transducer and is configured to be controlled by the respective ASIC 260. Other technologies, such as cMUT (capacitive micromachined ultrasonic transducers) or bulk piezoelectric transducers, can also be used instead of pMUT technology. In block diagram 200, only three TUS tiles 240 are shown to be connected. However, the TUS headband 120 may comprise a plurality of such tiles 240 (i.e. not limited to three TUS tiles 240) positioned in the TUS headband 120 such that they are configured to be arranged on different head parts of the user/subject 110. The TUS headband 120 may comprise a flexible cap configured to receive the TUS tiles 240 held in a predetermined position, with interconnection between the TUS tiles 240 achieved by flex circuits. The TUS tiles 240, the FPGA 210 and other electronic components, and physiological sensors may be mounted on the flex, which is disposed of within the cap.

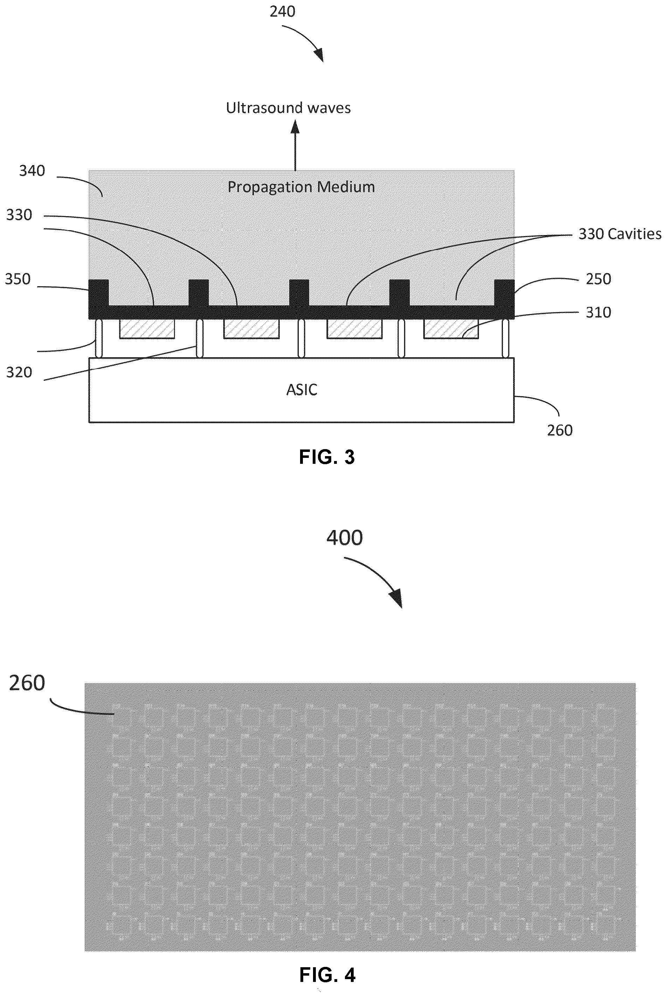

Fig. 3 shows an example of TUS tile 240 compatible with any embodiment of the invention in cross-section comprising pMUT MEMS array 250 (e.g. a matrix array) and integrated circuit configured as an ASIC 260. Referring to Fig. 3, MEMS devices 250 and ASIC 260 are connected using one or more vertical connections 320. Fig. 3 illustrates MEMS devices as silicon die 250 (optional feature), showing back-side of each MEMS etching at the top side and each MEMS device 250 being configured as (or comprising a) vibrating suspended membranes (called drums in this application) above a piezoelectric layer 310. Piezoelectric layer 310 is a sandwiched layer comprising a layer of piezoelectric material such as PZT (Lead Zirconate titanate), AIN (Aluminum Nitride), or AIScN (Aluminum Scandium Nitride) with metal electrodes above and beneath it. Fig. 3 also shows the propagation or coupling medium 340 (for example, a gel) that ensures proper acoustic coupling from the MEMS drums to the subject 110's scalp. In Fig. 3, subject 100's scalp would be positioned above the TUS tile 240, so the ultrasound waves labeled are directed into the head.

Referring back to Fig. 2, attached to the RT control 210 is a memory 220. The memory 220 contains a list of beam parameters. In an embodiment, the beam parameters are stored in a tabular format. Memory 220 can be an external DRAM, SRAM, EEPROM, memory within the FPGA, etc. RT control 210 reads beam parameters from memory 220 and uses it to control the operation of the TUS tiles 240. Beam parameters may be downloaded into the memory from off-device storage during a start-up procedure.

### Tiling or RX Chaining

As shown in Fig. 2, the TUS headband 120 may comprise multiple TUS tiles 240 that may be configured to be connected in a chain-connection. The ASIC 260 within each TUS tile 240 may be configured to accept a digital input stream 280 from an adjacent ASIC 260. The TUS headband device 120 may be configured (e.g. after appropriate delay and amplitude adjustment) to sum the digital input stream 280 with a data generated by the respective MEMS device 250 connected to the ASIC. This data generated by the MEMS devices 250 is also referred to a beamformed RX data. The TUS headband device 120 is configured such that the result of the summation is passed to the next tile's ASIC 260, and such that the output 285 of the final TUS tile 240 is sent to the RT control 210. The TUS headband 120 may be configured such that the TUS tiles may be selectively arranged in the chain-connection (e.g. when the TUS headband 120 operates in specific operating modes, such as in the channel mode and/or in the beam mode).

### EEG Subsystem

In an embodiment (as shown in Fig. 2), the TUS system 100 may comprise a physiological measurement subsystem 230 configured as an EEG subsystem 230. EEG subsystem 230 may be configured to communicate directly to the secure network device 130 in an embodiment. EEG subsystem 230 is used for monitoring the efficacy of treatment by monitoring EEG signals for biomarkers. Care is taken to ensure that there is no mutual interference between the EEG subsystem 230 and the operation of ultrasound with the TUS system 100. This is practical because the EEG data is very slow compared to the ultrasound data: it has a frequency range of around 1-100 Hz, while the ultrasound data is in the low MHz range. The EEG subsystem 230 may comprise a number of EEG channels in a range from 3 to 64. The operation of the EEG subsystem 230 involves a passive reception of scalp electrical signals of the user. This means it can be received directly secure network device 130 -e .g. by the microcontroller 290 (labeled µC)- and does not require specific connection to the RT control 210. The EEG subsystem 230 may comprise a signal conditioning circuitry (not shown), such as analog amplification and signal filtering, which may be implemented using commercially available devices. Alternatively, and advantageously as it reduces the package count, these electronics can be implemented in mixed-signal ASIC.

### LAYOUT OF INTEGRATED CIRCUITS

Fig. 4 shows a preferred layout 400 for the integrated circuits (each of which is preferably configured as a respective ASIC) of the plurality of TUS tiles 240. The plurality of ASICs 260 (i.e. one of each TUS tile 240) may be arranged in a grid layout 400. Fig. 4 shows an exemplary embodiment in which the ASICs 260 of the plurality of TUS tiles 240 are arranged in a 16x8 grid. Referring to Fig. 4, layout 400 comprises128 cells (wherein each cell represents an integrated circuit 260) arranged in a 16x8 grid (however it is noted that that this example merely represents a non-restricting embodiment of the invention). Further details of the configuration of each ASIC 260 cell are provided below in the specific description of the ASICs.

### MEMS

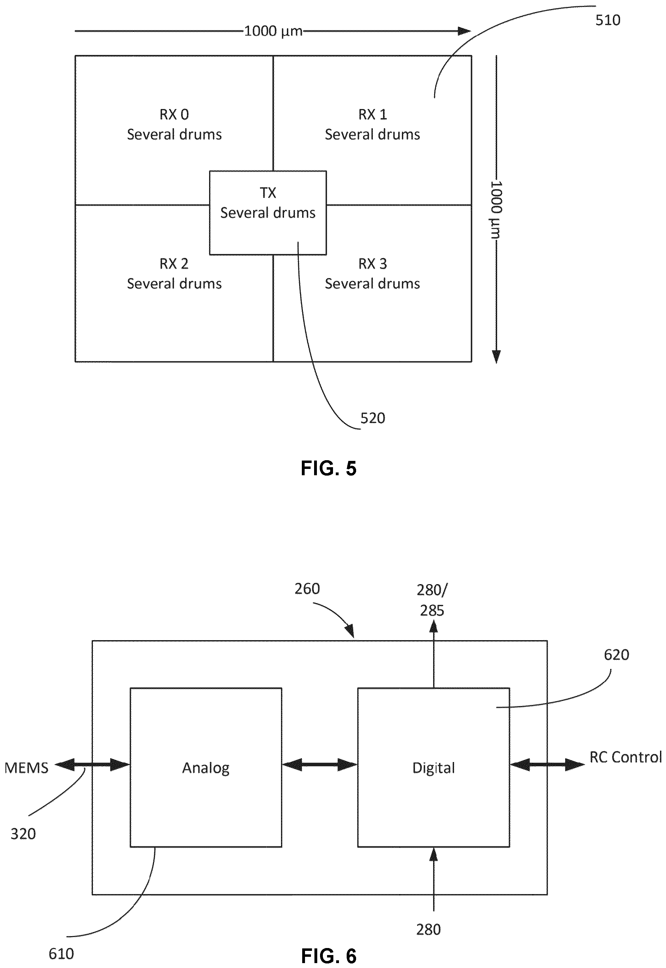

Fig. 5 shows an example of an internal layout of a MEMS die 250 transversally compatible with any of the embodiments of the invention. Fig. 5 shows a preferred configuration in which the MEMS die 250 die comprises four receive sections/portions 510 (independently identified as RX 0, RX 1, RX 2 and RX 3 in Fig. 5) with a centrally placed transmit section/portion 520 (also labelled as TX in Fig. 5). Separating transmit 520 and receive regions 510, as shown, simplifies the implementation in the ASIC 260 by removing the need for any transmit/receive switching logic. Each of the five sections is configured to be connected to the ASIC 260. A transmit portion 520 may correspond to an individual transducer element of a matrix array transducers, said individual element being configured as a transmitter element. Each receive portion 510 may correspond to a respective individual transducer element of a matrix array transducers, said individual element being configured as a receptor element. Each of the receive 510 regions may comprise several "drums", wherein a drum refers to an individual MEMS structure with vibrating suspended membranes (e.g. configured to be actuated by piezoelectric thin films). Similarly, the transmit 520 region may comprise several "drums". The number of drums for each receive section 510 and transmit section 520 may be fine-tuned in an implementation. Multiple drums may be configured to be connected parallelly on the MEMS die 250 to form the transmit 520 and receive 510 regions (also referred to as elements). In an embodiment, the receive sections 510 may be configured to operate at a different frequency from the transmit sections 520 (for example, RX frequency may be twice (2x) the TX frequency). The example layout shown in Fig. 5 allows the receive elements to be packed more finely to allow the receive sections 510 to operate at a higher frequency than the transmit sections 520. Other layouts with a different number of receive sections 510 and transmit sections 520 and in different geometrical arrangements are possible.

### ASIC

Fig. 6 shows an exemplary block diagram of an ASIC 260 cell compatible with the invention. Referring to Fig. 6, the ASIC 260 cell comprises an analog block 610 and a digital block 620. Preferably, the ASIC cell 260 may be configured such that a total surface/area of the ASIC cell 260 matches a surface/area of the respective MEMS cell 240 configured to be connected to said ASIC cell 260. However, some embodiments in which the ASIC cell 260 area does not match the MEMS cell 240, an interposer may be placed between them to enable connection from elements to ASIC receive inputs and transmit outputs.

### Analog Block

The analog block 610 is configured to be connected to a MEMS cell 250 by means of interconnects 320. Analog block 610 may be configured to convert an analog signal received from the MEMS cell 250 into a digital signal and to provide it to the digital block 620. The analog block 610 may comprise one or more analog receive blocks and an analog transmit block. Each analog receive block may be configured to receive analog functions/signals from a receive section 510 of the respective MEMS device 250 (e.g. the analog block 610 may be configured to have a number of analog receive blocks corresponding to a number of receive sections 510 comprised in the MEMS device 250 configured to be connected to the integrated circuit in which the analog block 610 is integrated). The analog transmit block may be configured to take input signals from the digital block 620 (e.g. with a correct timing and pulse train for one of the three types of operation in Table 1, namely, for operating in the channel mode, in the beam mode or in the stimulation mode) and convert them into an analog signal to drive MEMS devices/cells 250.

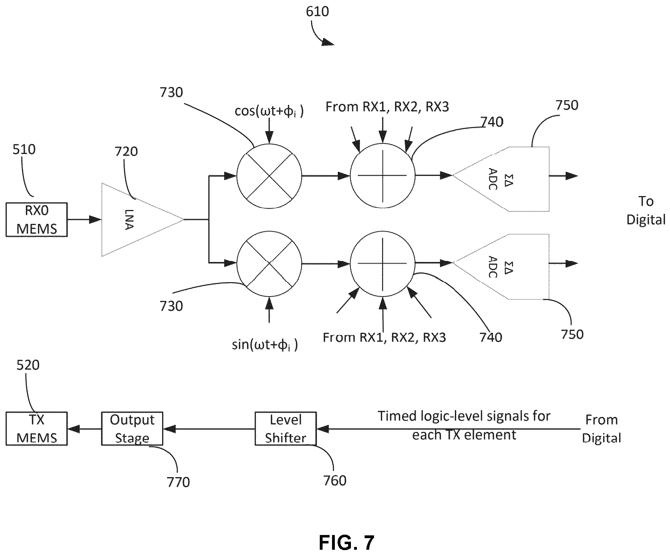

Fig. 7 shows two exemplary block diagrams compatible with the analog block 610 of an ASIC cell 260 shown in Fig. 6. The first diagram block of Fig. 7represents a analog receiving block (corresponding to the upper diagram in Fig. 7) of the analog block 610 configured to receive analog functions provided by a receiving section 510 of a MEMS cell 250 (which may correspond to the MEMS cell depicted in Fig. 5) and to provide an output to the digital block 620. In particular, this analog receiving block is configured to receive analog functions of the receiving section 510 labelled with the reference RX0 in Fig. 5. Although Fig. 7 only depicts a single analog receiving block, the analog block 610 may comprise as many analog receiving blocks as receiving sections have the respective MEMS cell 250. The analog receiving block shown in Fig. 7 comprises a LNAs (low-noise amplifiers) 720, two mixers 730, two analog summers 740, and 2 A/Ds 750 (Analog-to-digital converters or ADCs, sigma-delta ADCs may advantageously be used) for the receiver. The two mixers 740 are labelled in Fig. 7 with "cos(wt + Φ)" indicating that they may be performing frequency mixing with a local oscillator. Although not shown in Fig. 7, the analog block 610 may comprise Identical analog receiving blocks for the remaining receiving sections in the cell (e.g. those labeled as RX1, RX2, and RX3). Each analog receiving block may be configured to down-convert the analog functions received to baseband for the following reasons:
four channels can be combined after very simple phase adjustments to the local oscillator (e.g. in the mixers 730), so each analog receiving block requires only two ADCs instead of four. A factor f0/Δf decreases the me and Q ADC bandwidths compared with an RF approach, where Δf is the transducer's bandwidth and f0 is its center frequency.

A digital beam formation logic (i.e. a logic conducted by the secure network device 130 to generate a signal which may be configured to generate the skull compensation parameters and/or to obtain the anatomy characterization data, for example for characterizing/forming an output beam of the TUS tiles in any of the operation modes -e.g. in the stimulation mode, in the channel mode and/or in the beam mode-; in other words, the digital beam formation logic refers to the operation conducted by the secure network device to provide a signal specification for the output of the TUS tiles in each of the operating modes of the TUS system) is configured to service a number of data channels corresponding to the number of ASICs instead of having to service an independent digital signal for each receiving section of each ASIC 260. Thus, in the embodiment shown in Fig. 4 which comprises 128 integrated circuits configured as ASICs 260, the solution proposed by the invention merely manages 128 data channels (e.g. outputs of digital blocks 620) rather than 512.

Fig. 7 also shows an exemplary embodiment of an analog transmit block of an analog block 610 according to the invention. The analog transmit block is configured to receive input signals from the digital block 620 with a correct timing and to transmit them to the transmit section 520 of the respective MEMS cell 250. The analog transmit block may comprise a level shifter 760 (e.g. configured for shifting output voltage to an appropriate voltage for the transmit section 520) and an output stage 770. In an embodiment, output stage 770 may be configured to support a three-level output of +V, +V/2, and GND ("Ground") to supply symmetrical waveforms around +V/2. This ensures that the transmit section 520 actively returns to a resting voltage of +V/2. In an implementation, a "Ground" electrode may be configured to be common to all MEMS elements is held either at ground or at a bias voltage chosen to ensure that the field across the piezoelectric film never inverts and is always in the same direction as its poling. This optimizes the reliability of the film. Preferably, each ASIC 260 may comprise digital pads (e.g. connecting pins/points) located on two sides of the ASIC 260, which helps reduce the gap between transducers. This improves both transmit and receive performance.

In one implementation, operating frequencies vary between 0.5 to 3.0 MHz. In some embodiments, the TUS system may be configured such that the signals transmitted by each analog transmit block causes the respective transmit section 520 to provide an output (for each operating mode of the TUS system) including the following waveform characteristics:
- Channel mode operation: Short pulse, symmetrical 1-cycle or 2-cycle bursts at maximum output voltage to be used for skull characterization.
- Beam mode operation:
   ∘ For obtaining anatomy characterization data in the form of a volume grayscale image: short pulse centered at a lower frequency, with reception of 2^{nd} harmonic via, e.g., pulse inversion signaling.
   ∘ For mapping of cranial vessels: 4-8 cycle tone-burst centered at mid-band frequency.
- Stim Mode Operation: Long tone-burst (100-5000 cycles) centered between 0.5-2.0 MHz.

Typically, the waveforms' rise and fall times are configured to match and the delay quantization of transmit beamforming is around 75 ns.

### Digital Block

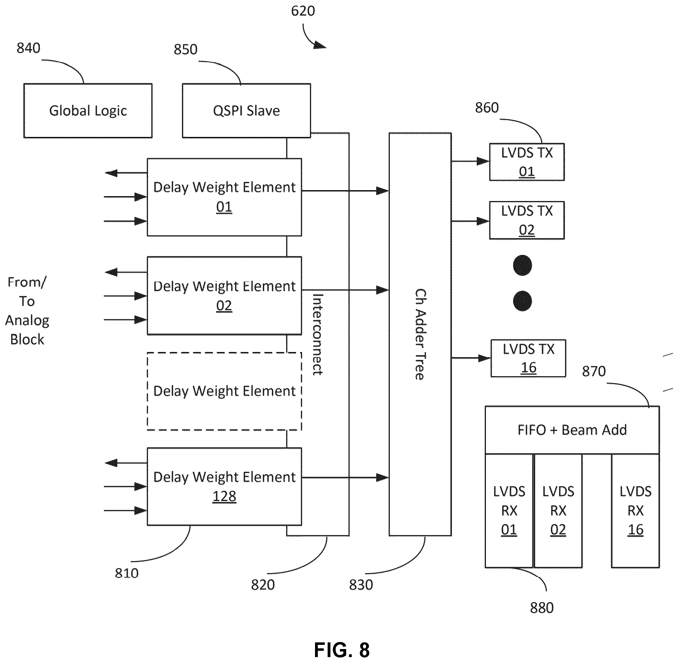

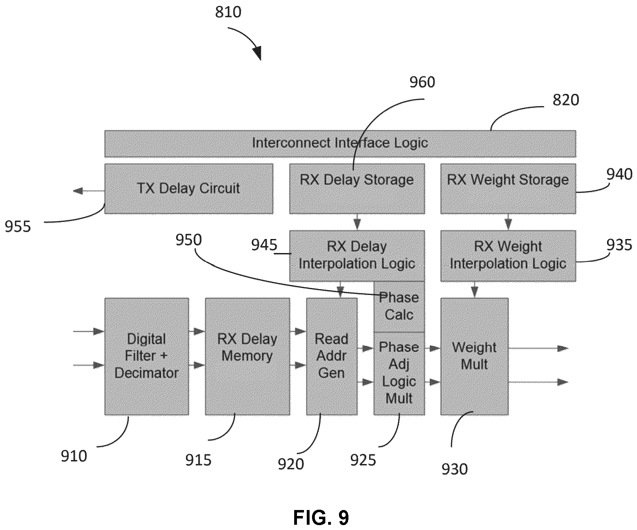

Fig. 8 shows an exemplary functional block diagram of the digital block 620 compatible with any integrated circuit 260 of the preceding embodiments. The digital block 620 may comprise a core configured as a Delay Weight Element (DWE) 810 subsystem. This subsystem, detailed further in Fig. 9, is configured to create correctly timed signals to be fed into the analog transmit block. The DWE may comprise a receiver section configured to receive data supplied to it from the baseband ADC 750 outputs of the respective analog receive block and carries out digital operations, resulting in the data related to beam formation (also referred to as beam formation data). Fig. 8 depicts an embodiment of a digital block 620 which exemplary comprises 128 (i.e. the same number of ASIC 260 used in the previous figures, although in other embodiments the number may differ) Delay Weight Elements 810, an Interconnect 820, a Channel Adder tree 830, a FIFO and Beam Adder 870, 16 LVDS transmit (TX) 860 & 16 LVDS receive (RX) 880 pairs, a QSPI slave interface 850, and a global logic block 840. The global logic block 840 (among other functions) may be configured to be responsible for taking the representations of beam direction and focal parameters supplied to it from the plurality of beam parameters (e.g. parameters of the Beam List, which may be located in the memory 220) and computing the low-level parameters stored in memories 960 and 940.

LVDS transmit (TX) 860 and LVDS receive (RX) 880 are used for tile chaining (i.e. when multiples TUS tiles 240 are connected in a previously described chain-connection) (e.g. in the channel operation mode and/or in the beam operation mode). The FIFO 870 is configured to delay a data received via the LVDS receive (RX) LVDS 880before being added to the TUS tile's data in Beam Add section 870 and sent to the next tile in the chain via LVDS TX 860.

The digital block 620 is configured to receive data (e.g. data measured by the at least one receive portion 510 of the respective MEMS device 250, preferably provided as an output of the ADC converters 750 of each analog receive block) i from the analog block 610, wherein the delay weight element 810 may be configured to apply a dynamic delay as a function of depth. The delay weight element 810 may be further configured to adjust, again as a function of depth, an amplitude of a channel data. After appropriately adjusting the amplitude and delay of the channel data, the resulting data may be fed to the channel adder tree 830 for summation. QSPI slave 850 may be configured to provide an interface to the RT control 210. The RT control 210 may be configured to use this interface to provide a beam list and other control information. The Global logic 840 may be configured to provide overall control to the digital block 620 and the interconnect 820 may be configured to provide on-chip interconnect between the various functional blocks.

### Delay Weight Element (DWE)

Fig. 9 shows an exemplary block diagram of a DWE 810 compatible with the one shown in Fig. 8. The DWE 810 shown in Fig. 9 comprises a TX Delay Circuit block 955 which is configured to implement delays (e.g. the beam parameters -e.g. of the beam list- may comprise transmit delays to be used for the TX Delay Circuit. These delays can be implemented using a memory (e.g. the delays may be located in the memory 220) or by other techniques.

The DWE is further configured to conduct a dynamic receive processing, which is more complex since the delays and weights change with depth. Values of delay and amplitude are required to be applied to the data at certain depths, wherein these values of delays and amplitudes are stored in memories 960 and 940. Logic in blocks 945 and 935 is configured to allow interpolated delays and weights to be applied at depths between those specified in memories 960 and 940.

The signal path for the delay weight element begins with the digital filter and decimator 910. The decimator 910 is configured to filter the ADC output to remove out-of-band noise, and to reduce the sampling rate to the lowest value consistent with adequate sampling of the baseband data. After this, a coarse delay is implemented through the write and read addresses of the delay memory 915. Generation of the read address, which varies with depth, is accomplished by block 920. Fractional delays may be implemented using an interpolator and logic 945. Further fine control of the signal phase may be implemented using the phase adjustment logic 925. After this processing, the received signals from a point target will be time-aligned across all channels and ready for summation. Before the summation, a multiplier 930 is configured to implement an apodize or weighting operation. This is driven by the weight interpolation logic 935 from data in memory 940.

### Closed Loop TUS Method

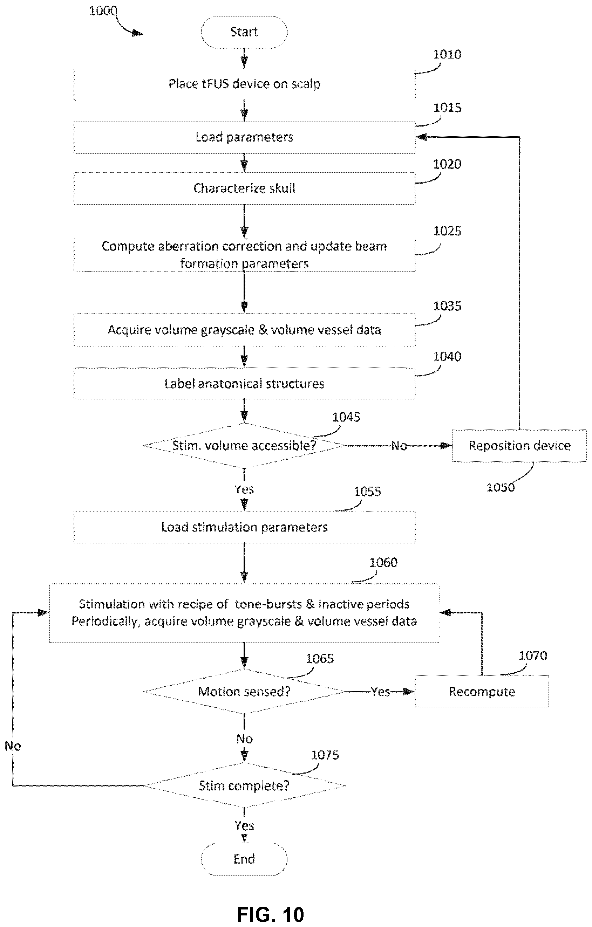

Fig. 10 is an exemplary method 1000 for optimizing an operation of TUS wearable 120 in a TUS system 100 according to the first aspect of the invention. Method 1000 can be performed using TUS system 100. Method 1000 is configured to correct skull aberrations, determine if the TUS device 120 is placed at a correct location, and if the device has shifted or moved while in use. All these functions synergistically contribute to provide an improved efficacy to the TUS system 100 (e.g. optimizing an energy required by the TUS system 100 to operate; e.g. improving targeting accuracy of the TUS system 100, leading to an increased safety for a user using the TUS system). Referring to Fig. 10, in operation 1010, wearable device 120 is placed on the scalp of subject 110.

In operation 1015, various beam parameters (e.g. such as those being part of the beam list), are loaded into the headband device 120. For example, an RT control 210 of the headband device may load the beam list from Memory 220 and provides it to ASICs 260 along with control information.

In operation 1020, device 120 is operated in "channel" mode for skull characterization. As described earlier, various characteristics of the human skull complicate ultrasound delivery to desired targets. In this operation, the method characterizes the skull's effects on ultrasound for each transducer element. In this operation, the TUS device's transducer elements may be configured to transmit a short pulse at a suitable center frequency. Received data may be captured with RX beamforming turned off, and the data are used to characterize the subject's skull.

In operation 1025, the received and transmitted data from operation 1020 are analyzed to determine the skull's effects, and appropriate correction parameters are computed for each transducer element. Beam formation parameters for all transducer elements are updated.

In operation 1035, the method maps and targets brain structures (or anatomy characterization) with the device operating in "Beam" mode. First, a volume grayscale image (2-D image) of the brain structures is acquired, followed by acquiring volume vessel data (blood flow) using Doppler processing.

In operation 1040, the method using pattern recognition, machine learning, or any suitable algorithms determines or labels the information acquired in Operation 1035 (brain structures and volume vessel data). This helps to determine if the device is placed correctly on the subject's scalp and, assuming adequate positioning, allows the stimulation to be targeted.

In operation 1045, the method determines if the stimulation target is accessible based on device 120's location on subject 110's scalp. If the target(s) are inaccessible, the following operation in the method is Operation 1050. If the target(s) are accessible, the following operation is Operation 1055.

In operation 1050, a message is sent to subject 110 from device 120 to reposition the device. For example, the message via secure network device 130 may be displayed on the app on subject device 140. The method proceeds to Operation 1015.

In operation 1055, stimulation parameters are loaded. In wellness mode, a variety of preset stimulation waveforms are available to a subject. In medical treatment mode, a clinician will specify the stimulation waveform and other parameters such as treatment length, burst length, time between bursts, etc. For example, the real-time control loads the beam list from Memory 220 and provides it to ASICs 260 along with control information. Steering and focusing information for all TX transducer elements is calculated. This includes a set of delays for each transducer element and, potentially, parameters enabling advanced beam formation involving plane wave transmission at differing angles.

In operation 1060, stimulation is started. Stimulation waveforms are long tone bursts (300 to 5000 cycles) centered at a suitable stimulation frequency, interspersed with inactive periods. Stimulation waveform parameters such as tone burst duration, pulse repetition frequency, acoustic frequency, amplitude intensity, etc., are determined by a clinician's treatment plan (or recipe). Device 120 ensures that no stimulation waveform parameters are beyond a safe range and that subject 110 cannot change stimulation waveform parameters to unsafe levels. Periodically, device 120 acquires grayscale and vessel data. For example, the device may acquire grayscale and vessel data after every 1/ (pulse repetition frequency) or a multiple of 1/ (pulse repetition frequency). Periodically acquiring grayscale and vessel data helps determine if headband 120 shifted while in use.

In operation 1065, using cross-correlation or other algorithms on sequentially acquired grayscale and vessel data, it is determined whether significant relative motion between the device or headband 120 and subject 110's scalp occurred. Significant relative motion between the device and the subject's scalp could cause ineffective stimulation since the stimulation beam may no longer be directed to the target tissue. If motion is sensed, the following operation is Operation 1070. If there was no significant movement, the following operation is Operation 1075.

In operation 1070, steering and focusing information for all TX transducer elements is recalculated. Method 1000 proceeds to operation 1060.

In operation 1075, Method 1000 checks if stimulation is complete. Stimulation could end due to an explicit command (from the subject) or because the prescribed NTB (number of tone bursts) is over. If the stimulation is incomplete, the method proceeds to operation 1060; otherwise, the method ends.

## Claims

1. A system (100) for ultrasound stimulation comprising:
a headband device (120) comprising one or more transcranial ultrasound, TUS, tiles (240), wherein each of the TUS tiles (240) further comprises a plurality of transducer elements (250) connected to a respective integrated circuit (260), wherein preferably the TUS tiles (240) are configured as a matrix of TUS tiles (240); and
a secure network device (130) configured to be in communication, preferably in wireless communication, with the headband device (120), the secure network device (130) being further configured for generating a signal specification and transmitting the signal specification to the headband device (120), wherein the headband device (120) is configured to operate in a stimulation mode in which the headband device (120) is configured to output a stimulation signal beam based on the signal specification via the one or more TUS tiles.

2. The system (100) of claim 1, wherein the headband device (120) further comprises at least one physiological measurement subsystem (230) configured to monitor an efficacy of the system (100) by measuring a physiological data related to a reaction in a user (110); wherein preferably:
each of the at least one physiological measurement subsystem (230) is configured to obtain measurements in the form of one of: an electroencephalogram, an electrooculogram, an electromyography or an electrocardiogram; and/or
the system (100) is configured to modify, preferably operating in a closed loop, a timing of the stimulation signal beam based on the physiological data measured by the at least one physiological measurement system (230).

3. The system (100) of any of the preceding claims, further comprising a secure network device (130) configured such that all communications with the system (100) are conducted through the secure network device (130), and wherein the secure network device (130) is configured to perform at least one of: authentication, encryption, tamper detection, and side-channel attack protection on behalf of the headband device.

4. The system (100) of any of the preceding claims, further comprising a real-time control circuit (210) and a memory (220) comprising a plurality of beam parameters compatible with one or more operations modes of the headband device (120), wherein the real-time control circuit (210) is coupled to the headband device (120)and is configured to read the beam parameters from the memory (220), wherein the TUS tiles (240) are configured to output the stimulation signal beam according to the beam parameters;
wherein preferably the plurality of beam parameters comprises a plurality of subsets of beam parameters, each subset of beam parameters being configured to characterize a respective operation mode of the headband device (120).

5. The system (100) of claim 4, wherein the real-time control circuit (210) is configured to cause the headband device (120) to operate in a channel mode in which the TUS tiles (240) are configured to obtain skull characterization data of a user by measuring an effect on ultrasound transmission caused by the skull of the user, wherein the real-time control circuit (210) is further configured to generate skull compensation parameters based on the skull characterization data for modifying an output of the TUS tiles (240), wherein preferably the skull compensation parameters are configured as time delay corrections and/or signal amplitude corrections.

6. The system (100) of claim 5, wherein the real-time control circuit (210) is configured to cause the headband device (120) to operate in a beam mode in which the headband device (120) is configured for mapping locations of brain structures of a user, such as blood flow locations and/or blood vessel locations, by means of the TUS tiles (240) to obtain anatomy characterization data;
wherein preferably the system (100) is further configured such that, when the headband device (120) operates in the stimulation mode, the real-time control circuit (210) causes the headband device (120) to adapt the stimulation signal beam based on the anatomy characterization data.

7. The system (100) of any of claims 5 or 6, further configured such that, when the headband device (120) operates in the channel mode or in the beam mode, the real-time control circuit (210) causes the TUS tiles (240) to communicate with an adjacent TUS tile (240), preferably in a chained-connection in which the TUS tiles (240) are sequentially connected to each other, such that the integrated circuit (260) of each TUS tile is configured to receive data from its respective plurality of transducers (250) and to transmit this data to the integrated circuit (260) of a next TUS tile (240) in the chained-connection, wherein the integrated circuit (260) of a final TUS tile (240) in the chained-connection is configured to provide a summation of the data generated by all the TUS tiles (240) and transmit this summation to the real-time control circuit (210).

8. The system (100) of claim 5 to 7, wherein the real-time control circuit (210) is configured to cause the headband device (210) to operate in a closed loop in which the headband device subsequently operates in the channel mode, in the beam mode and in the stimulation mode, such that the head band device is configured to:
continuously update the skull compensation data to generate updated skull compensation parameters in the channel mode;
continuously update the anatomy characterization data in the beam mode; and continuously adapt the stimulation signal beam based on the updated anatomy characterization data.

9. The system (100) of any of the preceding claims, wherein the plurality of transducer elements (250) of each TUS tile (240) are configured forming a micro-electromechanical system, MEMS, device (250) connected to the respective integrated circuit (260) of each TUS tile (240);
wherein preferably the plurality of transducer elements (250) of each MEMS device (250) comprises at least one of a Piezoelectric Micromachined Ultrasound Transducer, pMUT, or a Capacitive Micromachined Ultrasonic Transducers, cMUT, or a bulk piezoelectric transducer.

10. The system of claim 9, wherein each MEMS device (250) further comprises at least one receive portion (510) and a transmit portion (520), wherein the transmit portion (520) is electrically separated from the at least one receive portion (510); wherein preferably:
each of the at least one receive portion (510) and the transmit portion (520) are configured to be connected to the respective integrated circuit of the respective TUS tile (240); and/or
each MEMS device (250) comprises a plurality of receive portions (510), preferably four receive portions (510), and the transmit portion (520) is arranged centrally with respect to the four receive portions (510).

11. The system (100) of any of the preceding claims, wherein the integrated circuit (260) of each TUS tile (240) comprises an analog block (610) and a digital block (620); wherein preferably the integrated circuit (260) is configured as an Application-Specific integrated Circuit, ASIC.

12. The system (100) of claims 10 and 11, wherein the analog block (610) is configured to:
receive data measured by the at least one receive portion (510) of the respective MEMS device (250) and communicate said data to the digital block (620); and
receive output data from the digital block (620) and transmit said output data to the transmit portion (520) of the MEMS device (250), such that the transmit portion (520) provides an output of the respective TUS tile (240) further based on the output data.

13. The system (100) of any of the preceding claims, wherein the analog block (610) of each integrated circuit (260) comprises:
a respective analog receive block for each receive portion (510) of the MEMS device (250), each analog receive block comprising at least one of a LNA (720) configured to receive the data measured by the respective receive portion (510), two mixers (730), two summers (740), two sigma-delta ADC (750) in communication with the digital block (620); and/or
an analog transmit block configured to receive the output data from the digital block (620), the analog transmit block comprising a level shifter (760) and/or an output stage (770) configured to transmit the output data to the transmit section (520) of the respective MEMS device (250).

14. A method of optimizing the operation of a TUS system (100), which is preferably configured as a TUS system (100) according to any of the preceding claims, the method comprising:
placing a headband device (120) comprising a plurality of TUS tiles (240) on a subject scalp, the headband device (120) being part of a TUS system (100);
obtaining beam parameters from a memory (220), the beam parameters specifying an operational mode of the TUS tiles (240);
obtaining skull characterization data by measuring with the TUS tiles (240) an effect on ultrasound transmission caused by the skull of the subject;
calculating skull compensation parameters based on the skull characterization data;
adjusting the TUS parameters based on the skull compensation parameters;
generating a stimulation signal based on the adjusted TUS parameters; and
outputting the stimulation signal by means of the TUS tiles of the headband device (120).

15. The method of claim 14, further comprising obtaining anatomy characterization data by generating volume grayscale data or vessel data by means of the TUS tiles (240) to visualize a skull or blood vessel;
wherein preferably the method further comprises verifying positioning of the headband device (120) based on the volume grayscale data or vessel data;
wherein more preferably the method further comprises:
emitting the stimulation signal in response to verifying positioning of the headband device (120); and/or
determining, based on the volume grayscale data or vessel data, whether relative motion exceeding a threshold between the headband device (120) and the subject scalp occurred, and generating a message to reposition the headband device in response to the determination.
